# EUROPEAN PATENT SPECIFICATION

(11) **EP 3 804 767 B1**
(45) Date of publication and mention of the grant of the patent: **29.06.2022**
(21) Application number: 19921605.2
(22) Date of filing: 23.10.2019
(51) Int. Cl.: B05B 7/00, B05B 7/16, A61L 9/12, G09F 19/00, A61M 11/04

(54) **BOOSTER DEVICE FOR OLFACTORY DISPENSER**
VERSTÄRKERVORRICHTUNG FÜR OLFAKTORISCHE AUSGABEVORRICHTUNG
DISPOSITIF D'AMPLIFICATION POUR EMETTEUR OLFACTIF

(30) Priority: 28.03.2019 JP 2019063583
(43) Date of publication of application: 14.04.2021
(73) Proprietor: Aromajoin Corporation, Kyoto-city, Kyoto 612-8374 (JP)
(72) Inventor: KIM, Dong Wook, Kyoto-city, Kyoto 6128374 (JP); NAKANO, Kazuhiro, Kyoto-city, Kyoto 6128374 (JP)
(74) Representative: Treeby, Philip David William
(86) International application number: PCT/JP2019/041464
(87) International publication number: WO 2020/194818

(56) References cited:
- WO-A1-97/39779
- CN-U- 207 262 628
- JP-A- S 558 734
- JP-A- 2014 092 673
- JP-A- 2014 092 674
- JP-U- S50 124 193
- KR-B1- 101 951 841
- US-A1- 2010 243 754
- US-A1- 2016 021 325
- US-A1- 2017 119 919
- US-A1- 2017 360 980

## Description

### TECHNICAL FIELD

The present invention relates to an aroma display and, more specifically, to an improvement of an aroma display that is capable of emitting different scents by holding a plurality of aroma cartridges.

### BACKGROUND OF THE INVENTION

Human communication is done in various modes based on human senses. Most frequently used are visual and auditory communications. By contrast, olfaction or sense of smell, on which we rely considerably in our lives, is hardly used in communication. However, if the sense of smell can be utilized in addition to visual and auditory senses, communication would be more effective and various people would be able to share their experiences more profoundly.

Focusing on this point, recently, devices have been proposed which are used with audiovisual reproducing devices such as television receivers, personal computers and game machines for generating scents fitting for the time and place. In the present Specification, such a device that generates scents fitting for scenes will be referred to as an "aroma display".

Emission of a certain scent supposed to be fit for a certain scene would not be very effective if there is any lingering scent produced immediately before. Therefore, a desirable aroma display is one that can diffuse a desired scent in a certain limited scope at one time point and can blow out that scent and switch to another scent at another time point.

Without the capability to freely switch from one scent to another, an aroma display cannot make full use of scents. For this purpose, one possible approach is to prepare a plurality of cartridges (referred to as aroma cartridges), each containing a pre-selected scent emission source (referred to as a scent source), to load an aroma display with them, and emit the desired scent via the relevant cartridge.

A scent channel for carrying the scent from the scent source in the cartridge is formed in the cartridge, and a mechanism for feeding air into the cartridge at desired times is provided by the aroma display, so that the scent is emitted through the channel to the aroma display's exterior. Aside from the mechanism that emits scents from the cartridges, the aroma display also provides a mechanism for emitting air free of any scent component, having an air emission opening near the scent channels. To switch scents after one scent is emitted, the air-emitting mechanism emits a blast of air to blow out the scented air, and then another scent is emitted. Thus, scents can be switched at any timing, avoiding improper blending of scents.

Such a technique is disclosed in JP2014-092673A. The technique disclosed in JP2014-092673A is as described above. An opening is formed in the housing of an aroma display for emitting the scent to the outside. The scent channels, from their respective aroma cartridges and air emission openings, are all connected to this opening. Each aroma cartridge is releasably held in the housing of the aroma display. An opening for introducing air into the aroma cartridge is formed at a prescribed portion of each aroma cartridge. At the portion of the aroma display which corresponds to this opening when an aroma cartridge is held by the aroma display, a wind source containing a diaphragm with a piezoelectric element attached is mounted corresponding to each aroma cartridge. By applying AC voltage to the piezoelectric element, the diaphragm vibrates and introduces air into the aroma cartridge through a nozzle provided on the wind source and through the opening of the aroma cartridge. From the aroma cartridge that received the introduced air, scented air corresponding to the scent source therein is emitted to the outside through the scent channel.

Further, at the portion corresponding to the root of air emission opening of the aroma display, a wind source is provided as an air-emitting mechanism, which, having a structure similar to that of the wind sources attached to the aroma cartridges, can feed a larger amount of air, free of any scent, with stronger force to the air emission opening.

US 2017/119919 A1 discloses an aromatherapy nebulizing diffuser includes a bottom shell, a electric fan mounted in the bottom shell, a fluid container supported on the bottom shell, a cap caped on the fluid container, a oscillator mounted in the fluid container for oscillating a contained aromatic fluid into a fine mist of aromatic fluid droplets, and an air guide mounted in the fluid container and having a horizontally extended air outlet that mates with a curved inside wall portion of the cap to create a curved air flow path that guides the intake flow of air to strike against the inside wall of the cap and the container body and to further increase the air pressure for lifting the created fine mist of aromatic fluid droplets efficiently.

CN 207262628, US 2017/360980A1, KR 101951841 B1, US 2010/243754 A1 and US 2016/021325 A1 are also disclosed.

### SUMMARY OF INVENTION

### TECHNICAL PROBLEM

The aroma display having the above-described structure is capable of freely switching scents by selecting the wind source or sources to be operated. Further, by simultaneously operating the air-emitting mechanism for emitting scent-free air, it is possible to prevent the undesired blending of scents. The wind source to be operated and its timing may be controlled by sending an external command to the aroma display. Therefore, the aroma display can achieve magnificent effects of emitting desired scents appropriately by causing the desired wind sources to operate, for example, at desired timings in movies or animated films.

Because the conventional aroma display uses piezoelectric elements as wind sources in the mechanisms for emitting air and scent, it has the following drawbacks. While a wind source using a piezoelectric element can suppress the noise level, it can feed only a small amount of air. It poses no problem when the distance between the aroma display and a user is short, as in the case where the aroma display is used as an accessory of a PC. However, when it is used in a wider space, for example, when multiple people watch a TV, it is difficult to spread a scent far and wide or to dissipate the emitted scent immediately.

In order to solve such problems, it is possible to support the wind source of the air emission mechanism with a stronger fan. In that case, however, the fan noise would be problematic and the aroma display would be larger. Further, a larger fan leads to a problem in which it becomes difficult to maintain the emission of scent to a limited small area near the aroma display. A desirable aroma display is one that can emit a large volume of air to spread a scent far and wide, can dissipate the emitted scent, and can also emit a scent in a controlled manner within a limited space.

Therefore, an objective of the present invention is to make it possible to increase and decrease the volume of air flow that carries a scent emitted from an aroma cartridge.

### SOLUTION TO PROBLEM

According to a first aspect, the present invention provides a booster device of an aroma display that enhances the scent-emitting capability of the aroma display. The aroma display has a columnar shape having first and second surfaces and a side surface. The first surface has an emitting opening for emitting a scent. A wind source for emitting the scent to the outside of the aroma display is provided in the aroma display. The booster device includes: a housing accommodating the aroma display therein; and a fan provided in the housing. The housing includes a cylindrical outer housing having an air inlet for taking in air and a holding member holding the aroma display in the housing. The holding member holds the aroma display such that an air passage, through which air from the fan passes, is formed between the outer housing and the aroma display.

Preferably, both the aroma display and the outer housing have a central axis, and the holding member holds the aroma display such that central axes of the aroma display and the outer housing are parallel to each other.

More preferably, the holding member holds the aroma display such that the central axis of the aroma display is aligned with the central axis of the outer housing.

More preferably, the booster device for an aroma display further includes a narrowing member for narrowing the air flow generated by the fan, provided at an end portion of the outer housing on the side of the first surface of the aroma display.

Preferably, the booster device for an aroma display further includes a heating means provided in the air passage for heating air passing through the air passage.

More preferably, the booster device for an aroma display further includes a heat exchanging means provided in the outer housing for conducting heat exchange between the air passing through the air passage and air outside the outer housing.

More preferably, the heat exchanging means is provided to discharge heat of the air passing through the air passage to the outside of the outer housing and thereby to cool the air passing through the air passage.

Preferably, the heat exchanging means is provided to introduce heat of the air outside said outer housing into said outer housing to heat the air passing through said air passage.

More preferably, an opening is formed on a side surface of the outer housing; the heat exchanging means includes a Peltier module having first and second surfaces to contact respective objects of heat exchange; and the Peltier module is attached to the opening such that the first surface faces the air passage and the second surface faces outside of the outer housing.

More preferably, the booster device for an aroma display further includes a heat sink formed on the second surface of the Peltier module; the Peltier module has an upper edge same in length as an edge at the upper end of the opening, and the upper edge is in contact with the edge at the upper end of the opening; and a lower end side of the Peltier module is arranged at such a position that air flow generated by the fan is split to the air passage side and to the heat sink side by the Peltier module.

More preferably, the booster device for an aroma display further includes a heat insulating means arranged between the air passage and the aroma display.

More preferably, the heat insulating means is arranged between the air passage and at least that portion of the aroma display which holds a scent component.

The booster device of an aroma display may include a plurality of fans, and the plurality of fans may be arranged at equal intervals around the aroma display in the housing.

The fan may be provided in the housing between the second surface and the bottom surface of the housing.

The foregoing and other objects, features, aspects and advantages of the present invention will become more apparent from the following detailed description of the present invention when taken in conjunction with the accompanying drawings.

### BRIEF DESCRIPTION OF DRAWINGS

Fig. 1 is an oblique downwards perspective view of the aroma display used in an embodiment of the present invention.
Fig. 2 is an exploded, oblique downwards perspective view of the aroma display shown in Fig. 1.
Fig. 3 is a cross-sectional view take along the line 3-3 of the aroma display shown in Fig. 1.
Fig. 4 is an enlarged cross-sectional view of a micro blower.
Fig. 5 is a perspective view of the booster device of an aroma display in accordance with a first embodiment of the present invention.
Fig. 6 is an exploded perspective view of the booster device shown in Fig. 5.
Fig. 7 is a cross-sectional view taken along the line 7-7 of the booster device shown in Fig. 5.
Fig. 8 is a cross-sectional view taken along the line 8-8 of an upper case of the booster device shown in Fig. 7.
Fig. 9 is a cross-sectional view showing air flow in the booster device shown in Fig. 5.
Fig. 10 is an oblique downwards perspective view of the booster device of an aroma display in accordance with a second embodiment of the present invention.
Fig. 11 is an exploded perspective view of the booster device shown in Fig. 10.
Fig. 12 is a cross-sectional view taken along the line 12-12 of the booster device shown in Fig. 10.
Fig. 13 is a cross-sectional view showing air flow in the booster device shown in Fig. 10.
Fig. 14 is an enlarged cross-sectional view of a part of Fig. 13.
Fig. 15 is an oblique downwards perspective view of a booster device of an aroma display in accordance with a third embodiment of the present invention.
Fig. 16 is an exploded perspective view of the booster device shown in Fig. 15.
Fig. 17 is a cross-sectional view taken along the line 17-17 of the booster device shown in Fig. 15.

### DESCRIPTION OF EMBODIMENTS

In the following description and in the drawings, the same components are denoted by the same reference characters. Therefore, detailed description thereof will not be repeated. It is naturally understood that not every component of the embodiments described below is indispensable for the present invention. Some of the components may be omitted or replaced by other forms to affect the object of the present invention.

### [First Embodiment]

### <Structure>

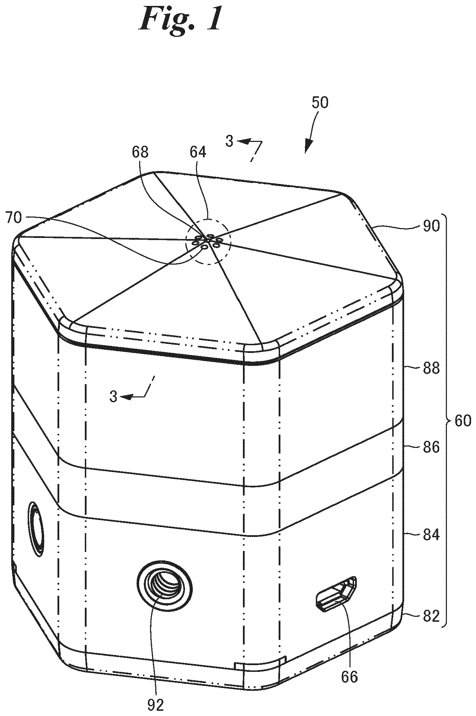

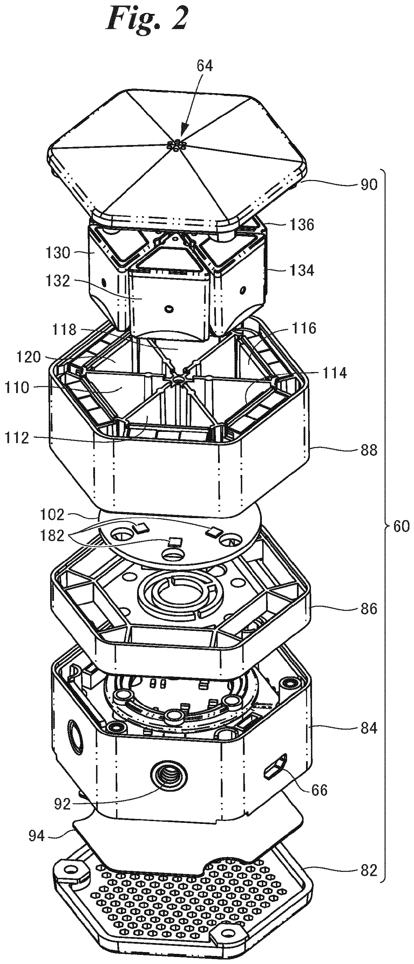

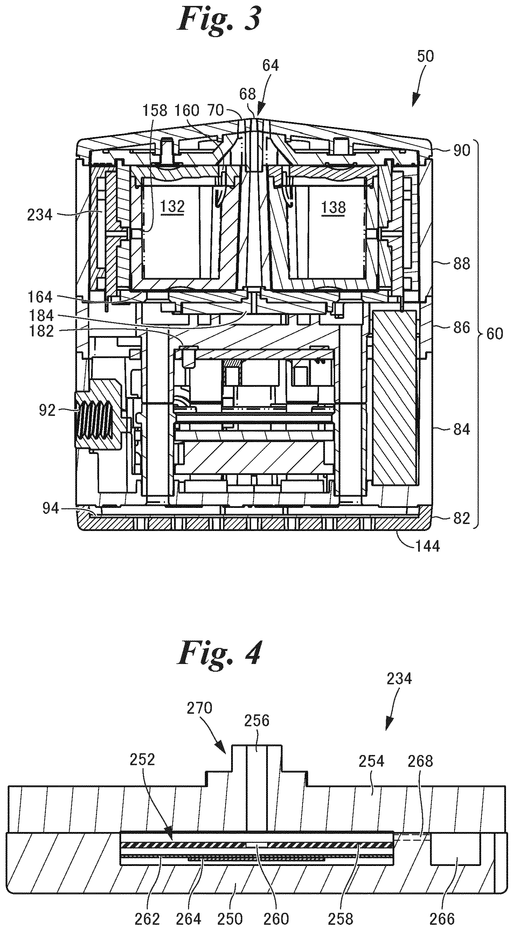

Fig. 1 shows an appearance of an aroma display 50 that is secured in a booster device of an aroma display in accordance with a first embodiment of the present invention. As shown in Fig. 1, aroma display 50 has a housing 60 that is a substantially hexagonal column with a central axis. Fig. 2 is an exploded perspective view of aroma display 50, and Fig. 3 is a cross-sectional view taken along the line 3-3 in Fig. 1 of aroma display 50.

Referring to Figs. 1 to 3, housing 60 includes: a base panel 82; a base body 84 arranged above the base panel 82 and attached to the base panel 82 by screws; a middle body 86 fitted to the base body 84 from above base body 84; an upper body 88 fitted to the top of the middle body 86, having a cartridge loading section opening upward, which may be loaded with six aroma cartridges; and an upper panel 90 fitted to the upper body 88 to close the upper opening of the upper body 88. By removing the upper panel 90, the opening of the upper body 88 is exposed, allowing a user to access the aroma cartridge loading section, to load or unload the loading section with aroma cartridges.

In the present embodiment, the upper body 88 has a slightly rounded hexagonal cross-section, and the cartridge loading sections 110, 112, 114, 116, 118 and 120 which may be loaded with six aroma cartridges 130, 132, ... 134, 136 and 138 (as to aroma cartridge 138, see Fig. 3) are formed inside the upper body 88. As a result, as shown in Fig. 2, aroma cartridges 130,... are held in the upper body 88. By way of example, on a bottom surface of the aroma cartridge 132, an NFC tag 164 (see Fig. 3) is attached, and at a nearby portion of the upper body 88, six NFC chips such as NFC chip 182 capable of wireless communication with the NFC tag 164 are provided. As can be understood from the description above, aroma display 50 has a substantially hexagonal column shape having an upper surface, a bottom surface and a side surface, with a central axis.

On a side surface of the base body 84, a screw hole 92 is formed as shown in Figs. 1 to 3, to which a tripod may be screwed in. The screw hole 92 is formed to meet a specific standard so that a tripod for a camera can be fitted.

Referring to Fig. 2, the base body 84 contains: a control board having mounted thereon a communication device for wireless communication to/from the outside, and a control circuitry for driving micro blowers, which will be described later; and a battery as the power source thereof. Between the middle body 86 and the upper body 88, an NFC chip stage 102 is provided, on which NFC chips 182 and the like communicating with NFC tags 164 provided at the bottoms of aroma cartridges 130, ... are mounted. Further, on the base panel 82, a large number of small holes are formed to take in air, used to generate power to blow out scented air from the aroma cartridges 130.... A filter 94 is provided between the base panel 82 and the base body 84 to filter out dust from the introduced air.

NFC tags 164 of aroma cartridges 130, ... store ID codes of the scents contained in respective aroma cartridges 130, .... When aroma display 50 is loaded with aroma cartridges 130, ..., NFC tags 164 communicate with corresponding NFC chips 182 and transmit ID codes of the scents contained in aroma cartridges 130, ... to NFC chips 182. NFC chips 182 transmit these ID codes to an external PC or the like through wireless communication. As a result, the external PC or the like recognizes the ID codes of respective scents of aroma cartridges 130, ..held by aroma display 50, and hence, it can control aroma display 50 such that scents are emitted in accordance with a program.

Referring to Figs. 1 to 3, at a central portion of an upper panel 90 of housing 60, there is an opening section 64. Particularly with reference to Figs. 1 and 3, at the center of the opening section 64, an opening 68 is formed, from which scent-free air from an air emission mechanism situated in housing 60 is emitted, and around this opening, six openings 70, ... are formed, from which scents are emitted from six aroma cartridges 130, ..., held by housing 60.

As described above, aroma display 50 can be mounted on a tripod for a camera. By mounting aroma display 50 on a small tripod that can be placed on one's desk, it becomes possible to place the aroma display 50 by the side of a PC, for example, with the opening section 64 of the aroma display 50 positioned very close to and directed to the user's face. This is effective to send desired scents to the user.

A micro USB connector 66 is provided on a side surface of the housing 60. In this embodiment, connector 66 is for feeding power to the aroma display 50 through a USB cable and for charging a built-in battery of the aroma display 50. Note that the USB cable does not provide external communication for the circuitries in the aroma display 50. The circuitry inside the aroma display 50 exchanges data with the outside through wireless communication. Naturally, the inner circuitry of the aroma display 50 may perform data communication with the outside through wired communication by the USB cable.

Particularly referring to Fig. 3, at that portion of the lower surface of upper body 88 which is above NFC chip stage 102, a micro blower 184 forming a mechanism for emitting scent-free air is fixed. Mechanisms including micro blower 184 for feeding air to the inside of aroma cartridges 130, ... will be described later.

Referring to Fig. 2, the upper body 88 has a substantially hexagonal cross-section as described above. The inner space of upper body 88 is divided into six aroma cartridge loading sections 110, 112, 114, 116, 118 and 120, each of which is a space having a regular triangle cross-section. Aroma cartridges 130, ... are respectively inserted into the cartridge loading sections 110, ....

Referring to Fig. 3, by way of example, aroma cartridge 132 has a triangular prism housing of which horizontal cross-section is substantially a regular triangle. At the portion of the upper surface of the housing which faces opening section 64 when the cartridge loading section is loaded with the aroma cartridge, an opening 160 is formed, which is connected to a hollow space inside the housing of aroma cartridge 132 for emitting scented air from the scent source sealed in the housing. At the portion of a side surface which is in contact with an inner surface of upper body 88, an air feeding opening 158 is formed for feeding air from an outside micro blower 234 to the inside of cartridge 132. On the lower surface of aroma cartridge 132, an NFC tag 164 is attached, for example, by means of a sticker, for near-field communication with an NFC chip 182.

At that portion of the inner side surface of upper body 88 at which air feeding opening 158 for feeding air into the aroma cartridge is positioned when respective cartridges 130,... are held by cartridge loading sections 110, micro blower 234 for feeding air into air feeding opening 158 is arranged. By driving micro blower 234, air is fed to the inside of aroma cartridge 132 and from the opening 160 for emitting scent of aroma cartridge 132 positioned near the center, scented air is emitted to the outside.

Micro blower 234 has the same structure as those disclosed in JP2014-092673A mentioned above. Referring to Fig. 4, specifically, the micro blower 234 has a case 250 with a blower chamber 252 as an inner space, and a cover member 254 with a nozzle 270 formed on its top surface, attached to case 250 so that it covers the top surface of case 250 to close the blower chamber 252. An air channel 256 is formed in nozzle 270, and air channel 256 opens into the upper portion of blower chamber 252.

Blower chamber 252 is divided into two upper and lower two portions by a partition plate 258. At a position immediately below air channel 256 of partition plate 258, an opening 260 is formed and registered with air channel 256.

In the lower space of blower chamber 252 divided by the partition plate 258, a diaphragm 262 formed of a thin metal plate with spring resiliency, and a piezoelectric element 264 bonded to the lower surface of diaphragm 262 are provided. By applying AC voltage to piezoelectric element 264, diaphragm 262 vibrates up and down and emits air through opening 260 to blower chamber 252 and further through air channel 256 to the outside. By positioning the micro blower 234 inside the upper body 88 such that nozzle 270 is positioned at the opening on the back side of the aroma cartridge, air can be introduced into the aroma cartridge 132 at a desired timing for a desired time period, and thus, scented air can be emitted from the aroma cartridge 132 and so on. It is noted that an air intake opening 266 is formed in case 250, and the air from intake opening 266 is fed through an air passageway 268 to blower chamber 252.

Each of the aroma cartridges other than the aroma cartridge 132 also has a micro blower 234 arranged for feeding air into the aroma cartridge through the opening on the back side. By operating these independently, it is possible to make a desired aroma cartridge emit the scented air at a desired timing for a desired time period. Micro blower 184 of the mechanism for emitting scent-free air shown in Fig. 3 also has the same structure as this micro blower, and hence, it can emit air at a desired timing for a desired time period from opening 68 shown in Figs. 1 to 3. Micro blower 184 is larger than other micro blowers 234 and, therefore, the amount of air emitted from opening 68 is far larger than micro blowers 234 such that micro blower 184 can easily dissipate any scent lingering near opening section 64 or carry a scent further.

It is noted, however, that micro blower 184 feeds air by the vibration of piezoelectric element, as shown in Fig. 4. While micro blower 184 has good response with a low level noise, basically, the amount of air that can be fed from micro blower 184 is structurally limited. Therefore, in order to spread a scent from the aroma cartridge to a wide space or to carry a scent afar, the air from micro blower 184 on its own is insufficient. Though replacing micro blower 184 with a larger fan is a possible solution, it becomes difficult to generate air flow of a certain volume in a quiet manner comparable to micro blower 184.

Therefore, a first embodiment of the present invention utilizes a booster that has a fan and surrounds aroma display 50 for spreading a scent from aroma display 50 far and wide or for quickly dissipating the scent.

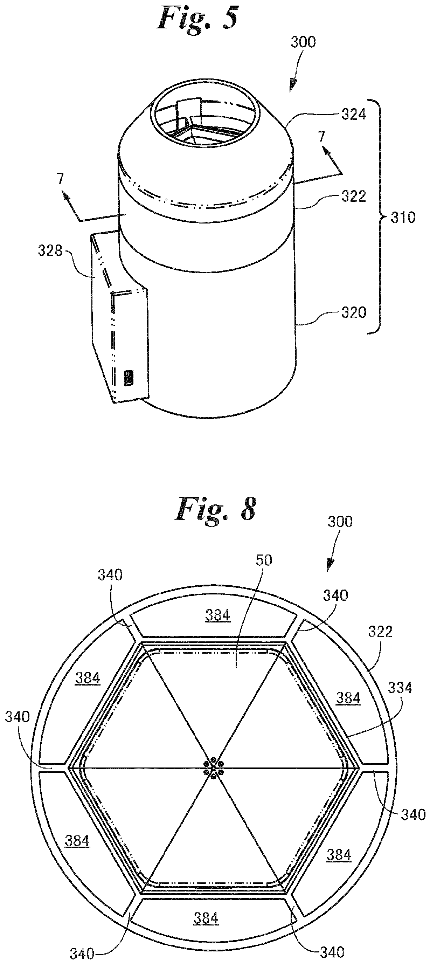

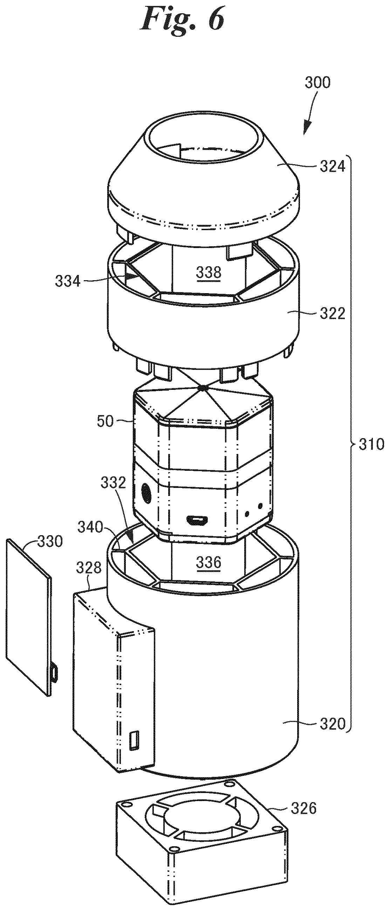

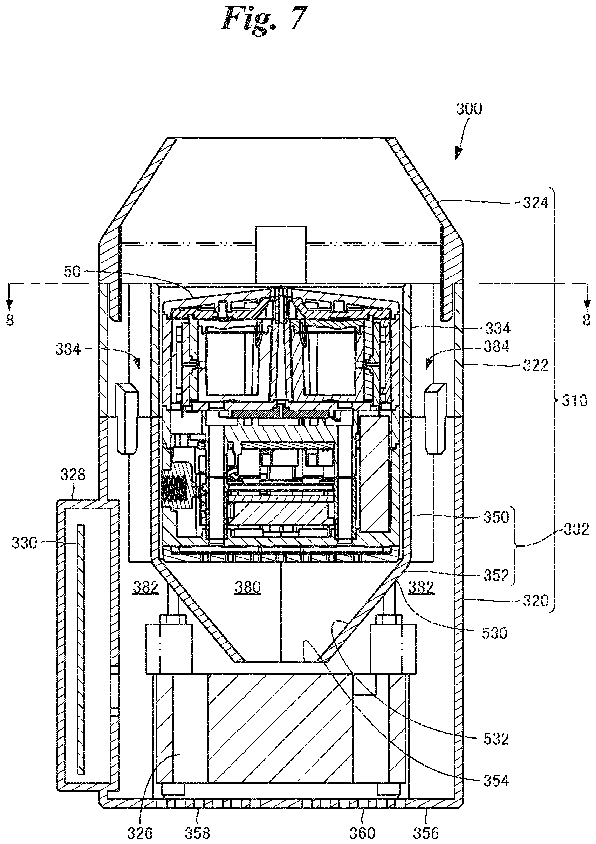

Fig. 5 shows an appearance of a booster 300 in accordance with the first embodiment of the present invention, and Fig. 6 is an exploded perspective view thereof. Fig. 7 is a cross-sectional view taken along the line 7-7 of the booster 300 shown in Fig. 5. Fig. 8 is a cross-sectional view taken along the line 8-8 of an upper case of booster 300 shown in Fig. 7.

Referring to Figs. 5 to 8, booster 300 has a columnar housing 310 of a circular cross-section with a prescribed central axis, in which aroma display 50 is contained. Housing 310 includes: a lower case 320 provided with a fan 326 therein and having a container portion 336 in which lower part of aroma display 50 is placed; an upper case 322 attached to an upper part of lower case 320 and having a container portion 338 in which upper part of aroma display 50 is placed; and a conical cap 324 having a circular cross-section attached to an upper part of upper case 322 where the circle diameter becomes smaller as a function of the distance from upper case 322. Cap 324 is open at upper and lower ends. This shape of cap 324 is, as will be described later, to attain the function of squeezing the air flow generated by fan 326 at the upper portion of upper case 322 and thereby to increase the velocity of air flow.

A partition member 332 having a hexagonal cross section defining container portion 336 and holding side surfaces of aroma display 50 is provided in lower case 320. Partition member 332 is fixed to the inside of lower case 320 by means of a member 340 that couples the lower half of each corner of the hexagon with the inner surface of lower case 320. As can be seen from Fig. 8, each of aroma display 50 and housing 310 has a central axis, and partition member 332 holds aroma display 50 such that the central axis of aroma display 50 and the central axis of housing 310 are parallel to and aligned with each other.

A board unit 328, in which a control board 330 is arranged, is formed at a portion on an outer periphery of lower case 320. As shown in Fig. 7, partition member 332 includes: a cylindrical portion 350 opened at upper and lower ends, having such a size and shape that just fits and holds aroma display 50 therein; and a section member 352 provided extending downward from the lower opening of cylindrical portion 350, having a conical shape opened at upper and lower ends, for dividing the air flow from fan 326 to the inside and outside of cylindrical portion 350. Section member 352 has outer and inner side surfaces 530 and 532 and an opening 354 at a lower end. Cross-section of partition member 332 has such a shape and size that aroma display 50 is a just fit for the inside of partition member 332. Section member 352 is tapered with its inner diameter becoming smaller downward and, therefore, aroma display 50 is supported by section member 352 at a boundary between the lower end of cylindrical portion 350 and the upper end of section member 352 and fixed inside the housing 310. Here, the central axis of aroma display 50 is aligned with the central axis of housing 310.

Upper case 322 also has a partition member 334 defining container portion 338. Referring to Figs. 7 and 8, cross-section of partition member 334 has a hexagonal shape that fits the housing of aroma display 50. Upper case 322 has its height selected such that, as is shown in Fig. 7, the top position of aroma display 50 is substantially even with the upper end of partition member 332 when aroma display 50 is held in housing 310. Upper ends of sides corresponding to respective vertexes of the hexagon of partition member 334 are connected to the inner surface of upper case 322 by means of member 340. As can be particularly well seen from Fig. 8, between the outer surface of partition member 334 and the inner surface of upper case 322, air passages 384 are formed, which will be described later.

Lower case 320 has a bottom plate 356 with a plurality of air inlets 358, ... 360 for taking in air. On bottom plate 356, fan 326 is mounted. Fan 326 is adapted to take air in from air inlets 358, ..., 360 formed in bottom plate 356, and to generate air flow in the direction of aroma display 50 in housing 310. In the present embodiment, the diameter of fan 326 is larger than the inner diameter of opening 354. There is some space between the lower end of section member 352 and the upper end of fan 326. In the present embodiment, fan 326 is driven by a DC-driven motor, and the maximum air flow rate of the fan is larger than that of micro blower 184 that serves as the air emission mechanism of aroma display 50. The objective of using fan 326 is to spread far and wide the scent emitted from aroma display 50 or to quickly dissipate any scent lingering rather widely around booster 300. Therefore, it is desirable that the maximum air flow rate of fan 326 is far larger than that of micro blower 184. By way of example, the maximum air flow of fan 326 is preferably 5 times, more preferably 10 times, and further preferably 50 times or more of the maximum air flow rate of micro blower 184. Naturally, it is desirable that the air flow rate of fan 326 is adjustable. If the size of fan 326 is too large, booster 300 becomes less portable and, therefore, the size of fan 326 is limited by itself. It is noted that fan 326 is provided inside booster 300 and, hence, noise caused by its operation can be kept low.

Particularly referring to Fig. 7, in booster 300 having the above-described structure, the inside of housing 310 is divided, by means of partition members 332 and 334, to an inner space 380 holding aroma display 50 and air passages 382 and 384 that are outside this space and formed as a ring-shaped space surrounding aroma display 50, and joined at the portion of cap 324. A plurality of openings are also formed at the bottom portion of aroma display 50, and micro blower 184 and so on of aroma display 50 take air in from space 380 and uses it for emitting scent from an aroma cartridge. Air passage 382 is formed by the outer side surface of section member 352 and the inner side surface of lower case 320. Air passage 384 is a channel further downstream of air passage 382 and, as shown in Figs. 7 and 8, it is formed by the outer side surface of partition member 334 and the inner side surface of upper case 322. Cross sections of air passages 382 and 384 are reduced around aroma display 50, accelerating air flow at this portion.

It is noted that cap 324 is detachable from upper case 322. By removing cap 324 from upper case 322, aroma display 50 can be removed from booster 300. Aroma display 50 can be mounted on booster 300 by the reverse procedure. If upper case 322 is made detachable from lower case 320, attachment/detachment of aroma display 50 becomes easier.

Control board 330 is connected by means of a cable, not shown, to a connector 66 and fan 326 of aroma display 50, feeding power to various electronic components in aroma display 50 and booster 300. Further, control board 330 drives fan 326 in accordance with an external control signal. In the present embodiment, aroma display 50 has a wireless communication function and using the wireless communication function, aroma display 50 directly communicates with an external control device. The present invention, however, is not limited to such an embodiment. Control board 330 may transmit a control signal to the inside of aroma display 50 through connector 66, or it may relay an output signal from aroma display 50 to an external control device.

### <Operation>

Aroma display 50 and booster 300 described above operate in the following manner.

First, a user inserts necessary aroma cartridges into aroma display 50. The user removes cap 324 from booster 300 and puts aroma display 50 into housing 310. Fig. 7 shows a cross section at this time of booster 300.

Assume that there is a scenario for a movie reproduced on a PC, for example, to emit a specific scent identified by the identifier at a specific scene for a specific time period in synchronization with the movie. Then the PC reads the scenario and reproduces the scenario in synch with the movie. Specifically, the PC transmits a command to the wireless communication device of the aroma display 50 such that a specific scent is emitted from the aroma cartridge with the designated identifier at the timing designated by the scenario. Receiving the command, the wireless communication device applies AC voltage at the designated timing for the designated time period to the micro blower 234 of the cartridge loading section loaded with the designated scent. As a result, the designated scent is emitted from the aroma display 50 at the timing and for the duration as designated by the scenario.

Here, it is possible to designate whether micro blower 184 inside aroma display 50 or fan 326 in housing 310 is to be operated simultaneously with the emission of a scent. When micro blower 184 is in operation, scent-free air is emitted to the outside of aroma display 50 from opening 68 shown in Figs. 1 and 3. Together with this air flow, a scent from an aroma cartridge flows out from cap 324 of booster 300. As a result, the user can feel the scent. In this case, however, the air flow generated by micro blower 184 is weak, and it does not reach very far. Therefore, the scent is felt in a close vicinity of booster 300 only.

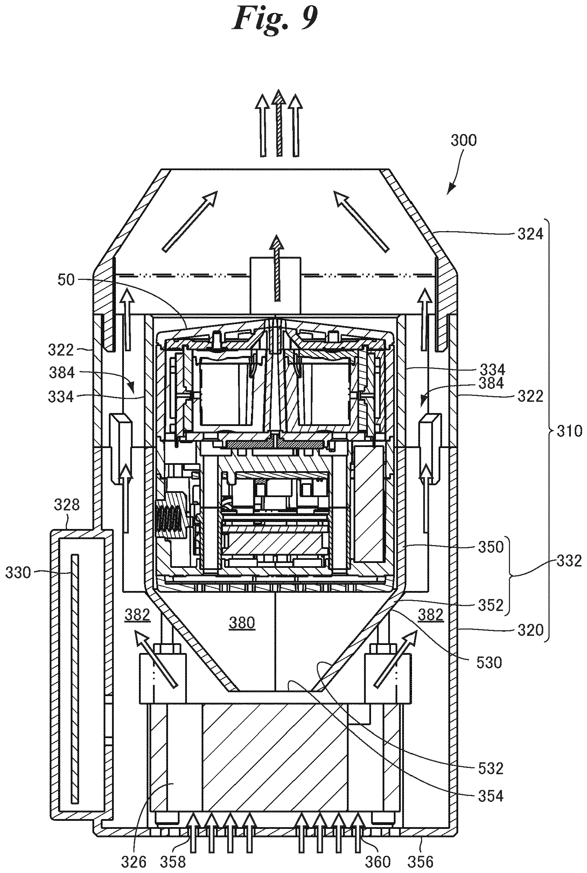

On the other hand, Fig. 9 shows air flow in booster 300 when it is fan 326 and not micro blower 184 that is driven. Referring to Fig. 9, the air flow generated by fan 326 is represented by white arrows, and the flow of scented air emitted by aroma display 50 is represented by hatched arrows. Referring to Fig. 9, fan 326 takes in air and, through inlets 358, ... 360 formed in bottom plate 356, air is introduced to the inside of housing 310. Since the inside of section member 352 is closed by aroma display 50, the air flow generated by fan 326 goes toward the ring-shaped air passage 382. This air flow is accelerated through air passages 382 and 384 around aroma display 50 and fed to cap 324. The scent let out from the tip end of aroma display 50 is emitted from housing 310 with velocity far higher than that caused by micro blower 184, because of the air flow that has passed through air passages 382 and 384; therefore, it reaches farther.

When the scent emitted from housing 310 is to be dissipated, the operation of various parts is the same as when the scent is emitted, except that no scent is emitted from aroma display 50. By emitting scent-free air to the outside of booster 300 using fan 326, not only the scent lingering near the booster 300 but also the scent lingering considerably far can be dissipated in a moment and blown away. Thus, the scent can be dissipated powerfully, and the possibility of unintentionally mixing with the next emitted scent is reduced.

When micro blower 184 is used, the air flow is weak and, therefore, only the scent lingering in the vicinity of booster 300 can be dissipated.

As described above, by the present embodiment, the scent generated by aroma display 50 can be carried far and wide by using fan 326. Further, it is possible to quickly dissipate the scent lingering in a wide range. It is also possible to carry only the scent-free air far. As a result, it becomes possible to emit a scent to a wide space and to prevent the mixing of scents when the scents are switched.

On the other hand, when aroma display 50 and booster 300 are used personally with a PC on one's desktop, it is unnecessary to use fan 326. Use of micro blower 184 is sufficient for personally enjoying a program with scents.

Further, according to this embodiment, aroma display 50 can freely be attached to/detached from booster 300. Therefore, for example, a plurality of aroma displays 50 loaded with different aroma cartridges may be switched easily for use. Further, it is also possible to take out aroma display 50 from booster 300 and to use the aroma display 50 by itself.

### [Second Embodiment]

### <Structure>

The aroma display 50 in accordance with the first embodiment described above enables emission of a scent to a wider space or to dissipate scent from a wider space as compared with a conventional aroma display. As a result, use of aroma display 50 can be widened from personal use to more general use by a larger number of people. There is still room for improvement to let users more fully enjoy the scents.

In a real life setting, a scent is felt not only by olfaction or sense of smell. In most cases, a scent is felt with temperature. By way of example, when one takes a sip of coffee, the aroma of coffee is felt with its warmth. On the contrary, one feels the scent of plants and trees with coolness in morning winds in the grass or in the forest.

The second embodiment has not only the function of emitting scents but also a function of heating or cooling the air containing scent, fit for the scene when the scent is presented.

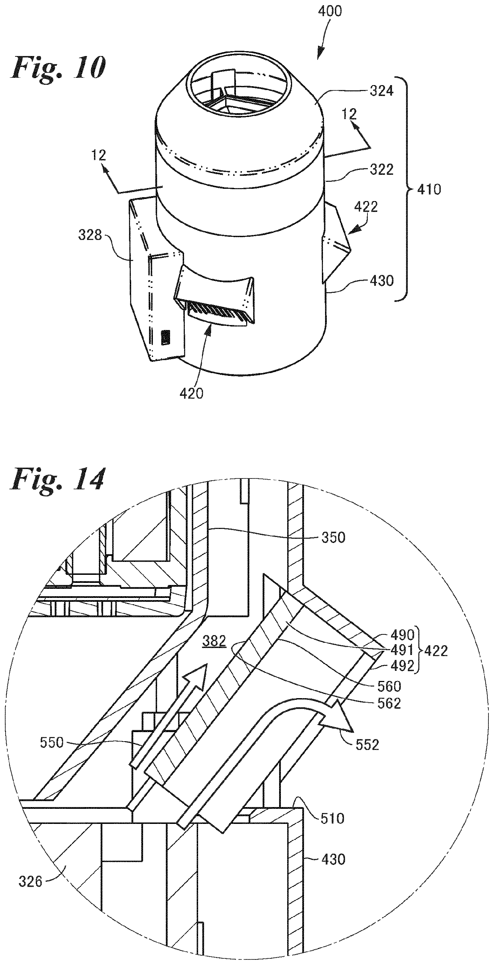

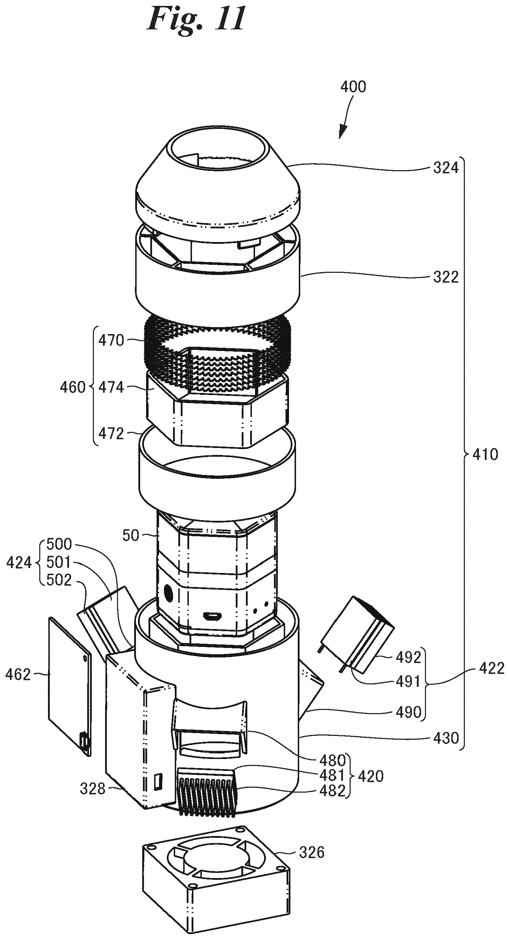

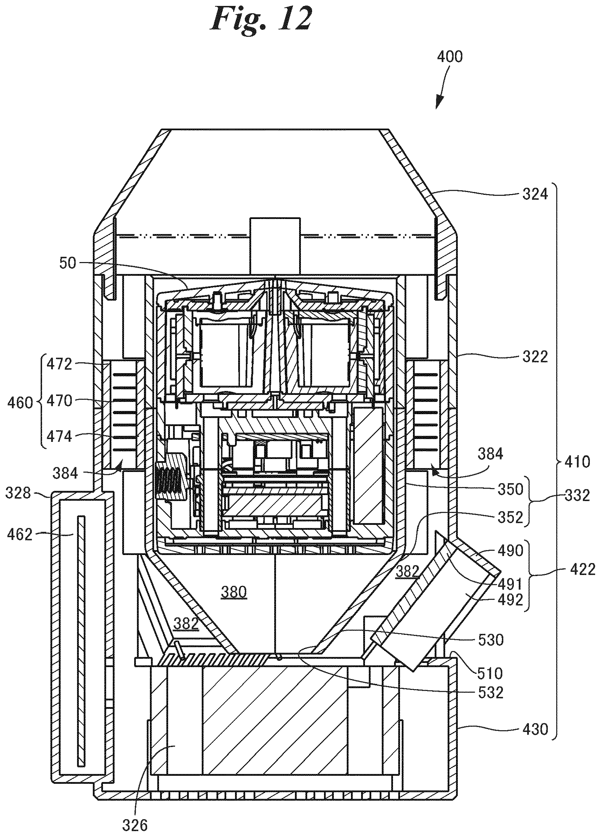

Fig. 10 shows an appearance of a booster 400 for an aroma display in accordance with the second embodiment. Fig. 11 is an exploded perspective view of booster 400. Fig. 12 is a cross-sectional view taken along the line 12-12 of Fig. 10. Referring to Figs. 10 to 12, booster 400 has a housing 410, of which appearance is different from that of housing 310 in accordance with the first embodiment. Housing 410 includes: a lower case 430 provided with a fan 326 therein and having a container portion in which lower part of aroma display 50 is placed, and having three heat exchanging units 420, 422 and 424 (heat exchanging unit 424 is not shown in Fig. 10) in addition to board unit 328 on its periphery; an upper case 322 attached to the upper portion of lower case 430; and a cap 324 attached to the upper portion of upper case 322.

Three heat exchanging units 420, 422 and 424 all have the same structure. Referring to Fig. 12, by way of example, heat exchanging unit 422 includes: a plate-shaped Peltier module 491 with a Peltier element, provided at an opening 510 opened on a side surface of lower case 320 with the heat absorbing (heat radiating) side surface facing the air passage 382 outside the section member 352; a heat sink 492 attached to the heat radiating (heat absorbing) side surface of Peltier module 491; and an overhanging portion 490 formed above opening 510 protruding like a visor and covering the upper surface of heat sink 492. Similarly, heat exchanging unit 420 includes an overhanging portion 480, a Peltier module 481 and a heat sink 482. Heat exchanging unit 424 includes an overhanging unit 500, a Peltier module 501 and a heat sink 502.

As is well known, Peltier module 491 is a plate-shaped semiconductor device and when a DC in a prescribed direction is applied, its heat absorbing surface cools an object that is in touch with the surface and radiates heat to the heat radiating surface. When the direction of current is reversed, the heat absorbing and radiating surfaces are switched. In other words, Peltier module 491 has a heat exchanging function. The width of Peltier module 491 is substantially equal to the width of opening 510, and the upper edge of Peltier module 491 is in contact with an upper edge of opening 510 in an air-tight manner. Peltier module 491 is attached to lower case 320 such that its lower edge is positioned further in from the side surface of lower case 320.

Therefore, the air flow generated by fan 326 is split by Peltier module 491 to the two sides thereof.

A heating unit 460 for heating air that passes through air passage 384 is provided in upper case 322. Heating unit 460 includes: a cylindrical outer heat insulator 472 provided to be in contact with an inner surface of upper case 322; a cylindrical inner heat insulator 474 provided to be in contact with an outer side surface of cylindrical portion 350; and a nichrome wire 470 wound a number of times around inner heat insulator 474, provided between outer and inner heat insulators 472 and 474. As is well known, nichrome wire 470 generates heat when a current is passed therethrough. The inner heat insulator 474 is to protect scent sources in aroma display 50 from the heated or cooled air in air passage 384. Scent generating characteristics of scent sources change when scent sources are heated or cooled. Therefore, inner heat insulator 474 is provided. The inner heat insulator 474 should be provided at such a position and range that protects from heating and cooling at least that portion of aroma display at which scent sources are positioned.

In board unit 328, in place of control board 330 of the first embodiment, a control board 462 is provided, which has a function of controlling Peltier module 491 and nichrome wire 470 in addition to the function of control board 330.

Except for this point, booster 400 has the same structure as that of booster 300 in accordance with the first embodiment. It is noted that when the direction of current is reversed, the heat radiating surface and the cooling surface of Peltier module 491 are switched. Therefore, it is possible to configure booster 400 such that the air is heated by reversing the direction of current fed to the Peltier module, rather than heating the air by using the nichrome wire. In that case, nichrome wire 470 is unnecessary. Further, it is also possible to use both Peltier module 491 and nichrome wire 470 to preheat the air by Peltier module 491 and additionally heat the air by nichrome wire 470. Such operation can be realized by controlling signals or currents applied externally to booster 400.

### <Operation>

Basically, the operation of booster 400 is the same as that of booster 300 in accordance with the first embodiment. Booster 400 is different from booster 300 in that it has the ability of heating or cooling the air generated by fan 326 and passing through air passages 382 and 384, in response to an external control signal.

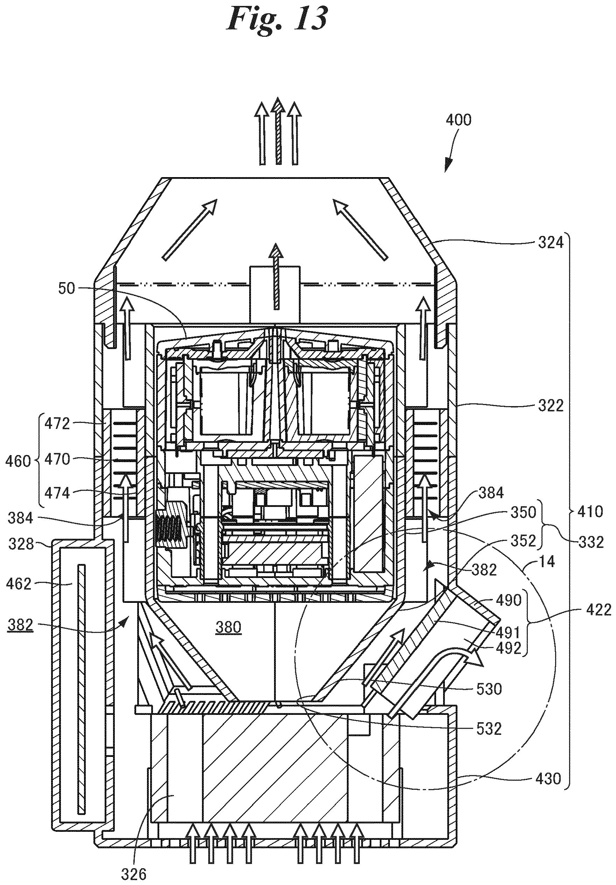

Fig. 13 shows air flow when booster 400 is in operation. In Fig. 13, white arrows indicate the flow of air generated by fan 326, and hatched arrows indicate the flow of scented air emitted from aroma display 50. Fig. 14 shows, in enlargement, the portion of Fig. 13 indicated by reference numeral 14. Basically, the flow of air in booster 400 is the same as that in booster 300 in accordance with the first embodiment. What is different is that the air passing through air passages 382 and 384 can be cooled or heated.

Particularly with reference to Fig. 14, a part of the air generated by fan 326 will be air flow 550 that flows through air passage 382 between section member 352 and heat absorbing surface 562 of Peltier module 491. The air flow 550 is deprived of its heat by heat absorbing surface 562 and cooled. Another part of air generated by fan 326 will be air flow 552 split from air flow 550 by Peltier module 491 and guided to the side of heat radiating surface 560 of Peltier module 491, which air flow deprives heat radiating surface 560 of heat and passes through heat sink 492, to be discharged outside from an opening of heat sink 492.

By way of example, by feeding DC to Peltier module 491 while driving fan 326, it is possible to cool the air flowing through air passages 382 and 384, and thereby carry and spread a scent emitted from aroma display 50 with cold air. While beautiful scenery of a mountain in a fresh morning is displayed on a PC screen, cold air with a fresh scent is spread, and a user will have a highly realistic sensation of a morning in the mountain. Here, part of the air flow from fan 326 is guided by Peltier module 491 to the side of heat sink 492, deprives radiating surface 560 of heat by means of heat sink 492, dissipates the heat into the surrounding air and thus enables effective operation of Peltier module 491.

Alternatively, it is possible to heat the air flowing through air passages 382 and 384 by feeding current to nichrome wire 470 without feeding any current to Peltier module 491. For example, when a scene of pouring coffee is to be displayed on a PC as described before, the air is heated by feeding current to nichrome wire 470 and a scent of coffee emitted from aroma display 50 is carried with heated air. As a result, the scent of coffee spreads with warm air and the user can feel the realistic sense of tasting a cup of coffee.

As described above, by the booster 400 in accordance with the present embodiment, in addition to simply spreading a scent, dissipating a scent at a desired timing, or switching scents, it is possible to adjust the temperature of air carrying the scent in accordance with the scent. As a result, communication using scents comes to have a stronger effect. If it is difficult to sufficiently heat only by nichrome wire 470, the current applied to Peltier module 491 may be reversed, so as to preheat the air passing through channel 384 and thereby to improve the heating effect. If Peltier module 491 has sufficient heating and cooling capacity, nichrome wire 470 may be omitted.

The invention has been described with reference to the embodiments. It is noted, however, that the invention is not limited to the embodiments above. For instance, in the embodiments above, the cross-sectional shape of aroma display 50 is substantially hexagonal and may be loaded with up to six aroma cartridges. Naturally, the shape of aroma display 50 is not limited to a hexagon. The cross-sectional shape of aroma display 50 may be determined independent of the number of aroma cartridges to be held therein, and it may have a shape of which number of corners match the number of cartridges to be held.

Further, in the embodiments above, aroma display 50 is detachable from booster 300 or 400. By adopting such a scheme, aroma display 50 may be removed from booster 300 or 400 and used by itself. When aroma display 50 is used by itself, it can be used personally on one's desktop in the conventional manner. The present invention, however, is not limited to such embodiments. Commercially, aroma display 50 and booster 300 or 400 may be sold separately or aroma display 50 and booster 300 or 400 may be sold as a set. When they are sold as a set, aroma display 50 and booster 300 or 400 may be packed separately, or packed with aroma display 50 arranged in booster 300 or 400.

Further, in the second embodiment, both the heating unit and the heat exchanging unit are used. The present invention, however, is not limited to such an embodiment. Only the heating unit or the heat exchanging unit may be provided in the booster. The numbers and positions of the heating units and the heat exchanging units are not limited to those on the booster 400 of the second embodiment. Positions of these units may be switched.

### [Third Embodiment]

### <Structure>

By the aroma display booster 400 in accordance with the second embodiment of the present invention, it becomes possible to spread a scent to or dissipate a scent from a wider area as compared with the conventional aroma display, and to attain highly realistic sensation of life scenes with scent, realizing more effective communication using scents. The structure for realizing such effects, however, is not limited to the structure described in the second embodiment. The third embodiment described in the following attains the effects similar to the booster 400 of the second embodiment by a different structure.

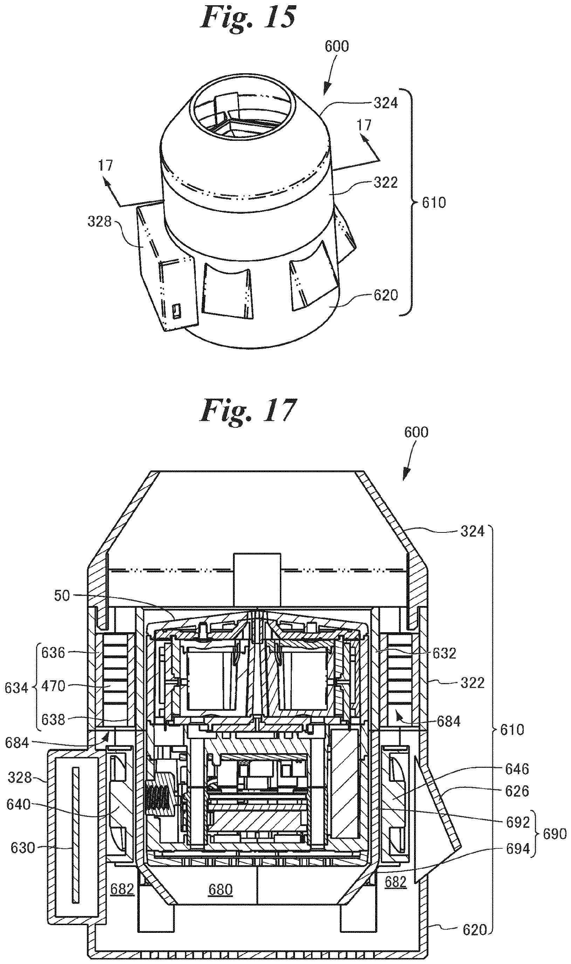

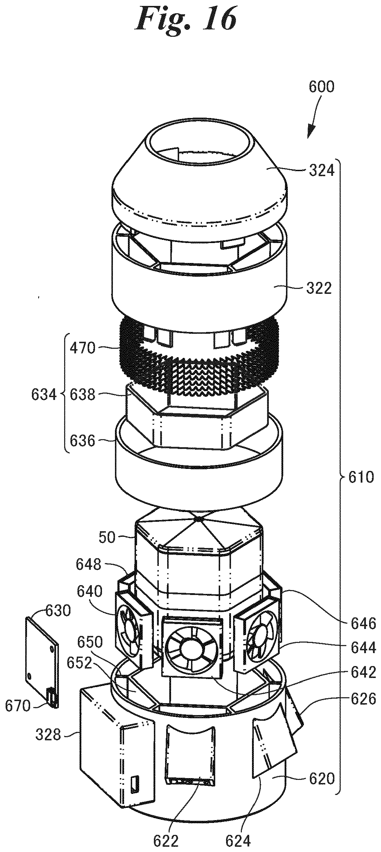

Fig. 15 shows an appearance of a booster 600 for an aroma display in accordance with the third embodiment of the present invention. Fig. 16 is an exploded perspective view of the booster 600. Fig. 17 is a cross sectional view taken along the line 17-17 of Fig. 15. Referring to Figs. 15 to 17, booster 600 includes a housing 610 of which appearance is different from housing 310 of booster 300 in accordance with the first embodiment. Housing 610 includes: a lower case 620 provided with small fans 640, 642, 644, 646,... 648 (see Fig. 16) therein in place of large fan 326 used in the second embodiment, in which a lower part of aroma display 50 is placed; an upper case 322 attached to the upper portion of lower case 620; and a cap 324 attached to the upper portion of upper case 322.

In the present embodiment, six small fans such as small fan 640 are arranged in six spaces formed by partition members 652 and 650 along an inner periphery of lower case 620. At those portions of lower case 620 which correspond to small fans 640, ... 648, air inlets 622, 624, 626, ... opened downward for feeding air are formed, except for a portion corresponding to small fan 640.

Referring to Fig. 16, in the present embodiment, small fans 640, 642, ... 648 are provided corresponding to respective aroma cartridges. The number of small fans 640 and so on, however, may be any number and not necessarily be the same as the number of aroma cartridges. Though it is preferred that the small fan 640 and other fans are arranged at equal intervals along the inner periphery of lower case 620, it is not a requirement of the present invention.

Referring to Fig. 17, in upper case 322, a partition member 632 similar to the partition member 334 (see Fig. 7) of the second embodiment is provided, dividing the inside of upper case 322 to a portion for accommodating aroma display 50 and an air passage 684.

Lower case 620 is also divided by a partition member 690 similar to the partition member 652 of the second embodiment to a portion accommodating aroma display 50 and a space 680 below, and an air passage 682. Similar to partition member 332 shown in Fig. 7, partition member 690 consists of a cylindrical portion 692 and a section member 694 that divides the space below aroma display 50 to an air passage 682 and a space 680 below aroma display 50.

Partition member 632 and cylindrical portion 692 have the same diameter and air passages 682 and 684 form one continuous air passage. An upper portion of air passage 682 is connected to an inner space of cap 324.

As in the second embodiment, in upper case 322, a heating unit 634 is provided for heating air that passes through air passage 684. Heating unit 634 includes: a cylindrical outer heat insulator 636 provided to be in contact with an inner surface of upper case 322; a cylindrical inner heat insulator 638 provided to be in contact with an outer side surface of partition member 632; and a nichrome wire 470 wound a number of times around inner heat insulator 638, provided between the outer and inner heat insulators 636 and 638.

In board unit 328, in place of control board 462 of the second embodiment, a control board 630 is provided, which has a function of controlling small fans 640, 642, ..., 648 and nichrome wire 470 in addition to the function of control board 330 of the first embodiment. A USB connector 670 for external connection is provided on control board 630.

Except for this point, booster 600 has the same structure as that of booster 300 in accordance with the first embodiment.

### <Operation>

Basically, the operation of booster 600 is the same as that of booster 300 in accordance with the first embodiment. Booster 600 is different from booster 300 in that in response to an external control signal, small fans 640, 642, 644, ... and so on take in air from the outside through air inlets 622, 624, 626, ... to generate air flow, which is fed to air passage 684.

The air flow when booster 600 is in operation is roughly the same as that shown in Fig. 9, and only difference is that small fans 640, 642, 644 and so forth take in open air through air inlets 622, 624, 626,... to generate air flow. Further, the present invention differs, as does the second embodiment, from the example shown in Fig. 9 in that the air passing through air passage 684 can be heated.

As described above, by the booster 600 in accordance with the present embodiment, in addition to simply spreading a scent, dissipating a scent at a desired timing, or switching scents, it is possible to heat the air carrying the scent in accordance with the scent. As a result, communication using scents will be more effective.

The embodiments as have been described here are mere examples and should not be interpreted as restrictive. The scope of the present invention is determined by each of the claims.

### INDUSTRIAL APPLICABILITY

The booster for an aroma display is applicable to a wide range of industries including advertisement using scents, promotion for aroma-related products such as perfumes, presentations using scents at movie theaters, with movie-reproducing apparatuses, with broadcast receivers such as televisions, at planetarium halls and schools, within the medical field for things such as treatment of anosmia, for presentation of educational materials for aroma therapists or perfumers, within the educational field for children or people with weak cognition of scents, and as a tool for better sleep environments to realize smooth sleep onset and refreshing awakening.

### REFERENCE SIGNS LIST

- 50: aroma display
- 60,310,410,610: housing
- 64: opening section
- 66: connector
- 68, 158, 184, 260, 354: openings
- 82: base panel
- 84: base body
- 86: middle body
- 88: upper body
- 90: upper panel
- 92: screw hole
- 94: filter
- 102: NFC chip stage
- 110, 112, 114, 116, 118, 120: cartridge loading sections
- 130, 132, 134, 136, 138, 140: aroma cartridges
- 164: NFC tag
- 182: NFC chip
- 184: micro blower
- 250: case
- 252: blower chamber
- 256: air channel
- 258: partition plate
- 262: diaphragm
- 264: piezoelectric element
- 266: air intake opening
- 270: nozzle
- 300, 400, 600: booster
- 320, 430, 620: lower case
- 322: upper case
- 324: cap
- 326: fan
- 328: board unit
- 330, 462, 630: control board
- 332, 334, 632, 690: partition member
- 336, 338: container portion
- 350, 692: cylindrical portion
- 352, 694: section member
- 356: bottom plate
- 358, 360, 622, 624, 626: air inlet
- 380,680: space
- 382, 384, 682, 684: air passage
- 420, 422, 424: heat exchanging unit
- 460, 634: heating unit
- 470: nichrome wire
- 472, 636: outer heat insulator
- 474, 638: inner heat insulator
- 480, 490, 500: overhanging portion
- 481, 491, 501: Peltier module
- 482, 492, 502: heat sink
- 510: opening
- 530: outer side surface
- 532: inner side surface
- 550: air flow
- 560: heat radiating surface
- 562: heat absorbing surface
- 640: small fan
- 670: USB connector

## Claims

1. A booster device for enhancing scent-emitting capability of an aroma display (50), wherein
said aroma display (50) has a columnar shape having first and second surfaces and a side surface, said first surface having an emitting opening for emitting a scent, and a wind source for emitting said scent to the outside of said aroma display is provided in said aroma display, maximum air flow generated by the wind source being equal to or smaller than a prescribed value;
said booster device comprising:
a housing (60) detachably accommodating said aroma display (50) therein; and
a fan (326) provided in said housing (60), maximum air flow generated by which is larger than said prescribed value; wherein
said housing (60) includes a cylindrical outer housing having a bottom surface with an air inlet (358) for taking in air and a cap (324) with an opening, and a holding member holding said aroma display (50) in said housing (60) such that said emitting opening of said aroma display faces said opening;
said holding member holds said aroma display (50) such that an air passage (382) is formed between said outer housing and said aroma display;
said fan (326) is arranged in said housing to blow out air to said air passage (382); and
said cap (324) has a narrowing member for narrowing the air flow generated by said fan, provided at an end portion of said outer housing on the side of said first surface of said aroma display (50).

2. The booster device for an aroma display according to claim 1, wherein each of said aroma display (50) and said outer housing has a central axis, and said holding member holds said aroma display (50) such that central axes of said aroma display (50) and said outer housing are parallel to each other.

3. The booster device for an aroma display (50) according to claim 2, wherein said holding member holds said aroma display (50) such that said central axis of said aroma display is aligned with said central axis of said outer housing.

4. The booster device for an aroma display (50) according to any of claims 1 to 3, further comprising a heating means (460) provided in said air passage (382) for heating air passing through said air passage (382).

5. The booster device for an aroma display (50) according to any of claims 1 to 4, further comprising a heat exchanging means (420) provided in said outer housing for conducting heat exchange between the air passing through said air passage (382) and air outside said outer housing.

6. The booster device for an aroma display (50) according to claim 5, wherein said heat exchanging means (420) is provided to discharge heat of the air passing through said air passage (382) to the outside of said outer housing and thereby to cool the air passing through said air passage (382).

7. The booster device for an aroma display (50) according to claim 5, wherein said heat exchanging means (420) is provided to introduce heat of the air outside said outer housing into said outer housing to heat the air passing through said air passage (382).

8. The booster device for an aroma display (50) according to claim 5, wherein
an opening is formed on a side surface of said outer housing;
said heat exchanging means (420) includes a Peltier module (481) having first and second surfaces to contact respective objects of heat exchange; and
said Peltier module (480) is attached to said opening such that said first surface faces said air passage and said second surface faces the outside of said outer housing.

9. The booster device for an aroma display (50) according to claim 8, further comprising: a heat sink (482) formed on said second surface of said Peltier module (481); wherein
said Peltier module (481) has an upper edge same in length as an edge at the upper end of said opening, and the upper edge is in contact with said edge at said upper end of said opening; and
a lower end side of said Peltier module (481) is arranged at such a position that air flow generated by said fan (326) is split to said air passage side and to said heat sink (482) side by said Peltier module (481).

10. The booster device for an aroma display (50) according to any of claims 1 to 9, further comprising a heat insulating means (472, 474) arranged between said air passage (326) and said aroma display (50).

11. The booster device for an aroma display (50) according to claim 10, wherein said heat insulating means (472, 474) is arranged between said air passage (326) and at least that portion of said aroma display (50) which holds a scent component (130).

12. The booster device for an aroma display (50) according to any of claims 1 to 11, comprising a plurality of said fans, and said plurality of fans are arranged at equal intervals along an inner circumference of said housing at a portion surrounding said aroma display (50).

13. The booster device for an aroma display (50) according to any of claims 1 to 11, wherein said fan is provided in said housing between said second surface of said aroma display (50) accommodated in said housing and said bottom surface of said housing.

## Patentansprüche

1. Verstärkungsvorrichtung zum Erhöhen der Duftemissionsfähigkeit eines Aromadisplays (50), wobei
das Aromadisplay (50) eine Säulenform mit einer ersten und einer zweiten Oberfläche und einer Seitenoberfläche aufweist, wobei die erste Oberfläche eine Emissionsöffnung zum Emittieren eines Dufts aufweist und eine Luftzugquelle zum Emittieren des Dufts nach außerhalb des Aromadisplays in dem Aromadisplay bereitgestellt ist, wobei der maximale von der Luftzugquelle erzeugte Luftstrom gleich oder kleiner als ein vorgegebener Wert ist;
wobei die Verstärkungsvorrichtung Folgendes umfasst:
ein Gehäuse (60), das das Aromadisplay (50) abnehmbar darin unterbringt; und
ein Gebläse (326), das in dem Gehäuse (60) bereitgestellt ist, wobei der maximale davon erzeugte Luftstrom größer als der vorgegebene Wert ist; wobei
das Gehäuse (60) Folgendes umfasst: ein zylindrisches äußeres Gehäuse, das eine untere Oberfläche mit einem Lufteinlass (358) zum Ansaugen von Luft und eine Kappe (324) mit einer Öffnung aufweist, und ein Haltelement, das das Aromadisplay (50) derart in dem Gehäuse (60) hält, dass die Emissionsöffnung des Aromadisplays der Öffnung zugewandt ist;
das Halteelement das Aromadisplay (50) derart hält, dass ein Luftkanal (382) zwischen dem äußeren Gehäuse und dem Aromadisplay gebildet wird;
das Gebläse (326) in dem Gehäuse angeordnet ist, um Luft zu dem Luftkanal (382) auszublasen; und
die Kappe (324) ein Verengungselement zum Verengen des von dem Gebläse erzeugten Luftstroms aufweist, das an einem Endabschnitt des äußeren Gehäuses auf der Seite der ersten Oberfläche des Aromadisplays (50) bereitgestellt ist.

2. Verstärkungsvorrichtung für ein Aromadisplay nach Anspruch 1, wobei das Aromadisplay (50) und das äußere Gehäuse jeweils eine Mittelachse aufweisen und das Halteelement das Aromadisplay (50) derart hält, dass die Mittelachsen des Aromadisplays (50) und des äußeren Gehäuses zueinander parallel sind.

3. Verstärkungsvorrichtung für ein Aromadisplay (50) nach Anspruch 2, wobei das Halteelement das Aromadisplay (50) derart hält, dass die Mittelachse des Aromadisplays und die Mittelachse des äußeren Gehäuses miteinander fluchten.

4. Verstärkungsvorrichtung für ein Aromadisplay (50) nach einem der Ansprüche 1 bis 3, ferner umfassend ein Heizmittel (460), das in dem Luftkanal (382) bereitgestellt ist, um durch den Luftkanal (382) gelangende Luft zu erwärmen.

5. Verstärkungsvorrichtung für ein Aromadisplay (50) nach einem der Ansprüche 1 bis 4, ferner umfassend ein Wärmeaustauschmittel (420), das in dem äußeren Gehäuse bereitgestellt ist, um einen Wärmeaustausch zwischen durch den Luftkanal (382) gelangender Luft und Luft außerhalb des äußeren Gehäuses vorzunehmen.

6. Verstärkungsvorrichtung für ein Aromadisplay (50) nach Anspruch 5, wobei das Wärmeaustauschmittel (420) bereitgestellt ist, um Wärme der durch den Luftkanal (382) gelangenden Luft nach außerhalb des äußeren Gehäuses abzulassen und dadurch die durch den Luftkanal (382) gelangende Luft zu kühlen.

7. Verstärkungsvorrichtung für ein Aromadisplay (50) nach Anspruch 5, wobei das Wärmeaustauschmittel (420) bereitgestellt ist, um Wärme der Luft außerhalb des äußeren Gehäuses in das äußere Gehäuse einzubringen, um die durch den Luftkanal (382) gelangende Luft zu erwärmen.

8. Verstärkungsvorrichtung für ein Aromadisplay (50) nach Anspruch 5, wobei
eine Öffnung auf einer Seitenoberfläche des äußeren Gehäuses gebildet ist;
das Wärmeaustauschmittel (420) ein Peltier-Modul (481) mit einer ersten und einer zweiten Oberfläche für den Kontakt mit jeweiligen Wärmeaustauschgegenständen umfasst; und
das Peltier-Modul (480) derart an der Öffnung angebracht ist, dass die erste Oberfläche dem Luftkanal zugewandt ist und die zweite Oberfläche dem Äußeren des äußeren Gehäuses zugewandt ist.

9. Verstärkungsvorrichtung für ein Aromadisplay (50) nach Anspruch 8, ferner umfassend: eine Wärmesenke (482), die auf der zweiten Oberfläche des Peltier-Moduls (481) gebildet ist; wobei
das Peltier-Modul (481) eine Oberkante mit derselben Länge wie eine Kante an dem oberen Ende der Öffnung aufweist und sich die Oberkante mit der Kante an dem oberen Ende der Öffnung in Kontakt befindet; und
eine untere Endseite des Peltier-Moduls (481) in einer derartigen Position angeordnet ist, dass ein von dem Gebläse (326) erzeugter Luftstrom von dem Peltier-Modul (481) auf die Luftkanalseite und die Wärmesenke(482)-Seite aufgeteilt wird.

10. Verstärkungsvorrichtung für ein Aromadisplay (50) nach einem der Ansprüche 1 bis 9, ferner umfassend ein Wärmedämmungsmittel (472, 474), das zwischen dem Luftkanal (326) und dem Aromadisplay (50) angeordnet ist.

11. Verstärkungsvorrichtung für ein Aromadisplay (50) nach Anspruch 10, wobei das Wärmedämmungsmittel (472, 474) zwischen dem Luftkanal (326) und mindestens demjenigen Abschnitt des Aromadisplays (50), der eine Duftkomponente (130) hält, angeordnet ist.

12. Verstärkungsvorrichtung für ein Aromadisplay (50) nach einem der Ansprüche 1 bis 11, umfassend eine Vielzahl der Gebläse, und wobei die Vielzahl von Gebläsen in gleichen Abständen entlang eines Innenumfangs des Gehäuses an einem das Aromadisplay (50) umgebenden Abschnitt angeordnet sind.

13. Verstärkungsvorrichtung für ein Aromadisplay (50) nach einem der Ansprüche 1 bis 11, wobei das Gebläse in dem Gehäuse zwischen der zweiten Oberfläche des in dem Gehäuse untergebrachten Aromadisplays (50) und der unteren Oberfläche des Gehäuses bereitgestellt ist.

## Revendications

1. Dispositif d'amplification servant à augmenter la capacité d'émission de parfum d'un dispositif de présentation d'arômes (50), dans lequel
ledit dispositif de présentation d'arômes (50) a une forme de colonne ayant des première et deuxième surfaces et une surface latérale, ladite première surface ayant une ouverture d'émission servant à émettre un parfum, et une source de vent servant à émettre ledit parfum vers l'extérieur dudit dispositif de présentation d'arômes est mise en œuvre dans ledit dispositif de présentation d'arômes, un flux d'air maximum généré par la source de vent étant égal ou inférieur à une valeur prescrite ;
ledit dispositif d'amplification comportant :
un boîtier (60) logeant de manière détachable ledit dispositif de présentation d'arômes (50) dans celui-ci ; et
un ventilateur (326) mis en œuvre dans ledit boîtier (60), dont le flux d'air maximum généré par celui-ci est supérieur à ladite valeur prescrite ; dans lequel
ledit boîtier (60) comprend un boîtier extérieur cylindrique ayant une surface inférieure avec une entrée d'air (358) à des fins d'aspiration d'air et un capot (324) avec une ouverture, et un élément de retenue servant à retenir ledit dispositif de présentation d'arômes (50) dans ledit boîtier (60) de telle sorte que ladite ouverture d'émission dudit dispositif de présentation d'arômes est orientée vers ladite ouverture ;
ledit élément de retenue retient ledit dispositif de présentation d'arômes (50) de telle sorte qu'un passage d'air (382) est formé entre ledit boîtier extérieur et ledit dispositif de présentation d'arômes ;
ledit ventilateur (326) est agencé dans ledit boîtier pour faire souffler de l'air jusqu'audit passage d'air (382) ; et
ledit capot (324) a un élément de rétrécissement servant à rétrécir le flux d'air généré par ledit ventilateur, mis en œuvre au niveau d'une partie d'extrémité dudit boîtier extérieur sur le côté de ladite première surface dudit dispositif de présentation d'arômes (50).

2. Dispositif d'amplification pour un dispositif de présentation d'arômes selon la revendication 1, dans lequel chacun parmi ledit dispositif de présentation d'arômes (50) et ledit boîtier extérieur a un axe central, et ledit élément de retenue retient ledit dispositif de présentation d'arômes (50) de telle sorte que les axes centraux dudit dispositif de présentation d'arômes (50) et dudit boîtier extérieur sont parallèles l'un par rapport à l'autre.

3. Dispositif d'amplification pour un dispositif de présentation d'arômes (50) selon la revendication 2, dans lequel ledit élément de retenue retient ledit dispositif de présentation d'arômes (50) de telle sorte que ledit axe central dudit dispositif de présentation d'arômes est aligné sur ledit axe central dudit boîtier extérieur.

4. Dispositif d'amplification pour un dispositif de présentation d'arômes (50) selon l'une quelconque des revendications 1 à 3, comportant par ailleurs un moyen de chauffage (460) mis en œuvre dans ledit passage d'air (382) pour chauffer l'air passant au travers dudit passage d'air (382).

5. Dispositif d'amplification pour un dispositif de présentation d'arômes (50) selon l'une quelconque des revendications 1 à 4, comportant par ailleurs un moyen d'échange de chaleur (420) mis en œuvre dans ledit boîtier extérieur pour effectuer un échange de chaleur entre l'air passant au travers dudit passage d'air (382) et l'air à l'extérieur dudit boîtier extérieur.

6. Dispositif d'amplification pour un dispositif de présentation d'arômes (50) selon la revendication 5, dans lequel ledit moyen d'échange de chaleur (420) est mis en œuvre pour décharger de la chaleur de l'air passant au travers dudit passage d'air (382) vers l'extérieur dudit boîtier extérieur et pour de ce fait refroidir l'air passant au travers dudit passage d'air (382).

7. Dispositif d'amplification pour un dispositif de présentation d'arômes (50) selon la revendication 5, dans lequel ledit moyen d'échange de chaleur (420) est mis en œuvre pour introduire la chaleur de l'air à l'extérieur dudit boîtier extérieur jusque dans ledit boîtier extérieur pour chauffer l'air passant au travers dudit passage d'air (382).

8. Dispositif d'amplification pour un dispositif de présentation d'arômes (50) selon la revendication 5, dans lequel
une ouverture est formée sur une surface latérale dudit boîtier extérieur ;
ledit moyen d'échange de chaleur (420) comprend un module Peltier (481) ayant des première et deuxième surfaces à des fins de mise en contact avec des objets respectifs d'échange de chaleur ; et
ledit module Peltier (480) est attaché à ladite ouverture de telle sorte que ladite première surface est orientée vers ledit passage d'air et ladite deuxième surface est orientée vers l'extérieur dudit boîtier extérieur.

9. Dispositif d'amplification pour un dispositif de présentation d'arômes (50) selon la revendication 8, comportant par ailleurs : une source de froid (482) formée sur ladite deuxième surface du module Peltier (481) ; dans lequel
ledit module Peltier (481) a un bord supérieur identique en longueur à un bord au niveau de l'extrémité supérieure de ladite ouverture, et le bord supérieur est en contact avec ledit bord au niveau de ladite extrémité supérieure de ladite ouverture ; et
un côté d'extrémité inférieure dudit module Peltier (481) est agencé au niveau d'une position telle que le flux d'air généré par ledit ventilateur (326) est divisé vers ledit côté passage d'air et vers ledit côté source de froid (482) par ledit module Peltier (481).

10. Dispositif d'amplification pour un dispositif de présentation d'arômes (50) selon l'une quelconque des revendications 1 à 9, comportant par ailleurs un moyen calorifuge (472, 474) agencé entre ledit passage d'air (326) et ledit dispositif de présentation d'arômes (50).

11. Dispositif d'amplification pour un dispositif de présentation d'arômes (50) selon la revendication 10, dans lequel ledit moyen calorifuge (472, 474) est agencé entre ledit passage d'air (326) et au moins cette partie dudit dispositif de présentation d'arômes (50) qui retient un composant de parfum (130).

12. Dispositif d'amplification pour un dispositif de présentation d'arômes (50) selon l'une quelconque des revendications 1 à 11, comportant une pluralité desdits ventilateurs, et les ventilateurs de ladite pluralité de ventilateurs sont agencés selon des intervalles équidistants le long d'une circonférence intérieure dudit boîtier au niveau d'une partie entourant ledit dispositif de présentation d'arômes (50).

13. Dispositif d'amplification pour un dispositif de présentation d'arômes (50) selon l'une quelconque des revendications 1 à 11, dans lequel ledit ventilateur est mis en œuvre dans ledit boîtier entre ladite deuxième surface dudit dispositif de présentation d'arômes (50) logé dans ledit boîtier et ladite surface inférieure dudit boîtier.
